# EUROPEAN PATENT SPECIFICATION

(11) **EP 3 175 238 B1**
(45) Date of publication and mention of the grant of the patent: **25.08.2021**
(21) Application number: 15757340.3
(22) Date of filing: 27.07.2015
(51) Int. Cl.: A61K 35/20, G01N 33/50, A23C 7/04, A23L 33/00, A23C 9/142

(54) **DEVICES AND KITS FOR PRODUCTION OF PERSONALIZED, CONCENTRATED HUMAN MILK COMPOSITIONS, AND METHODS OF MAKING AND USING SAME**
VORRICHTUNGEN UND KITS ZUR HERSTELLUNG VON PERSONALISIERTEN, KONZENTRIERTEN MENSCHLICHEN MILCHZUSAMMENSETZUNGEN UND VERFAHREN ZUR HERSTELLUNG UND VERWENDUNG DAVON
DISPOSITIFS ET KITS DE PRODUCTION DE COMPOSITIONS LAIT HUMAIN CONCENTRÉ PERSONNALISÉ, ET PROCÉDÉS DE FABRICATION ET D'UTILISATION ASSOCIÉS

(30) Priority: 28.07.2014 US 201462029593 P
(43) Date of publication of application: 07.06.2017
(73) Proprietor: Société des Produits Nestlé S.A., 1800 Vevey (CH)
(72) Inventor: VERDI, Lesley Keane, Chester 07930 (US)
(74) Representative: Gagliardi, Tatiana
(86) International application number: PCT/IB2015/055676
(87) International publication number: WO 2016/016796

(56) References cited:
- EP-A1- 1 637 880
- US-A1- 2007 005 006
- MEIER PAULA P ET AL: "Accuracy of a user-friendly centrifuge for measuring creamatocrits on mothers' milk in the clinical setting.", BREASTFEEDING MEDICINE : THE OFFICIAL JOURNAL OF THE ACADEMY OF BREASTFEEDING MEDICINE SUMMER 2006, vol. 1, no. 2, July 2006 (2006-07), pages 79-87, XP002745131, ISSN: 1556-8342
- C W SAUER ET AL: "Human milk macronutrient analysis using point-of-care near-infrared spectrophotometry", JOURNAL OF PERINATOLOGY, vol. 31, no. 5, 1 May 2011 (2011-05-01), pages 339-343, XP055216650, ISSN: 0743-8346, DOI: 10.1038/jp.2010.123
- ITABASHI K ET AL: "Fortified preterm human milk for very low birth weight infants", EARLY HUMAN DEVELOPMENT, SHANNON, IR, vol. 29, no. 1-3, 1 June 1992 (1992-06-01) , pages 339-343, XP023146082, ISSN: 0378-3782, DOI: 10.1016/0378-3782(92)90189-N [retrieved on 1992-06-01]
- None

## Description

### BACKGROUND

Premature babies and low-birth-weight infants often need more concentrated nutrition due to their smaller, less developed digestive systems. Current strategies in the Neonatal Intensive Care Unit (NICU) include the use of cow milk-based human milk fortifier (HMF), which is added to human milk. Cow milk-based HMF provides significantly more protein and fat than human milk alone.

However, experts concur that delivery of species-specific nutrition to these infants is best, as it mitigates the risks of introducing allergens and as it provides valuable microflora and micronutrients needed by the species. Recent advancements have been made that allow for the production of human milk-based HMF from multiple donor milk supplies. However, there are some risks of transmitting disease when donor milk is used, so the human donor milk has to be sterilized and therefore may not have as many valuable microflora or micronutrients therein. EP 1 637 880 relates to a method for analysing and treating human milk to be fed to an infant. It furthermore relates to a system for processing results of nutritional analysis of a sample of collected human milk.

Therefore, there is a need in the art for new and improved methods of providing human milk fortifiers for premature and low-birth-weight infants that overcome the disadvantages and defects of the prior art, including the uses of cow milk-based HMF as well as human donor milk-based HMF. It is to such methods for producing and using human milk fortifiers, and especially personalized human milk fortifiers specifically produced from breast milk obtained from an individual infant's mother, as well as kits and devices related thereto, that the presently disclosed inventive concept(s) is directed.

### BRIEF DESCRIPTION OF THE DRAWINGS

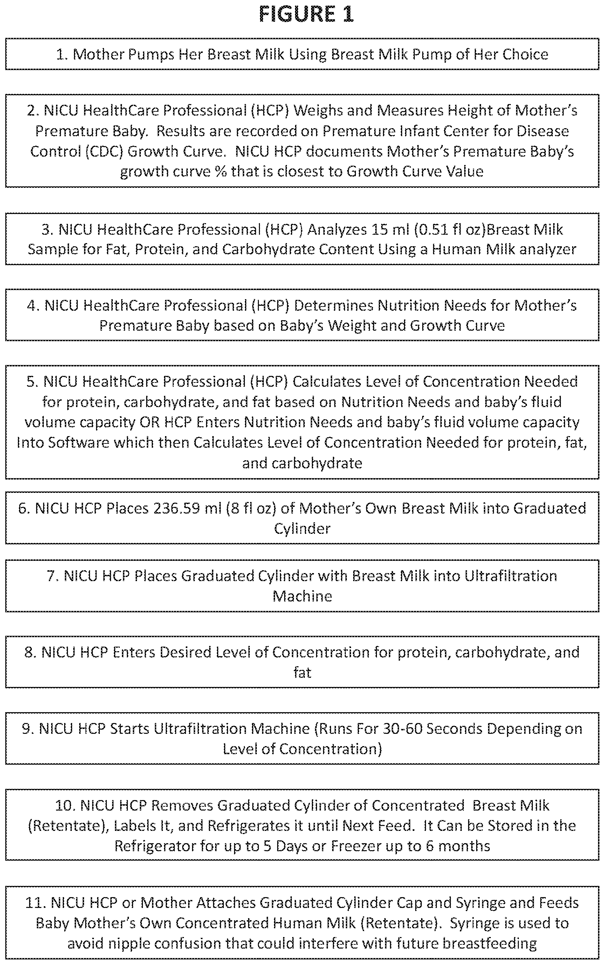

Figure 1 is a schematic flow chart illustrating one non-limiting example of a method constructed in accordance with the presently disclosed inventive concept(s).

### SUMMARY OF THE INVENTION

The present invention relates to a kit for producing a personalized, concentrated human milk composition for an infant, the kit comprising:
at least one analyzer for determining a concentration of at least one nutrient and/or at least one micronutrient present in the expressed breast milk from the mother;
at least one selective concentration device capable of concentrating at least one nutrient and/or at least one micronutrient present in expressed breast milk from a mother of the infant and wherein the selective concentration device is a selective ultrafiltration device;
at least one graduated container capable of receiving the expressed breast milk and capable of measuring a volume of the expressed breast milk, the at least one graduated container being capable of association with the selective concentration device for concentration of the breast milk disposed therein to produce the personalized, concentrated human milk composition;
and at least one device that calculates a desired level of concentration of the breast milk based upon a concentration of at least one nutrient and/or at least one micronutrient present in the expressed breast milk and at least one infant factor selected from the group consisting of the infant's height, the infant's weight, the infant's nutritional needs, the infant's fluid volume capacity, the infant's growth curve, and combinations thereof and wherein the device calculates a desired level of concentration of the breast milk based upon a concentration of each of a plurality of nutrients and/or micronutrients present in the expressed breast milk and a plurality of infant factors, and
wherein the calculated level of concentration is defined as:
   (i) a desired concentration of one or more of the plurality of nutrients and/or micronutrients; and/or
   (ii) a desired ratio of two of the nutrients/micronutrients to one another.

In one aspect the present invention relates to a method of producing a personalized, concentrated human milk composition for an infant, the method comprising the steps of:
(a) obtaining expressed breast milk from a mother of an infant;
(b) analyzing a portion of the expressed breast milk to determine a concentration of at least one nutrient and/or at least one micronutrient present therein;
(c) calculating a desired level of concentration of the expressed breast milk based upon the concentration of the at least one nutrient or micronutrient present in the expressed breast milk and at least one infant factor selected from the group consisting of the infant's height, the infant's weight, the infant's nutritional needs, the infant's fluid volume capacity, the infant's growth curve, and combinations thereof; and
(d) exposing the expressed breast milk to a selective concentration device which is a selective ultrafiltration device; that concentrates the at least one nutrient and/or the at least one micronutrient analyzed in step (b) to the desired level of concentration calculated in step (c), thereby producing the personalized, concentrated human milk composition;
And wherein, in step (b), a concentration of a plurality of nutrients and/or micronutrients present is the expressed breast milk is determined, and wherein, in step (d), the selective concentration device concentrates the plurality of nutrients and/or micronutrients
wherein preferably the desired level of concentration calculated in step (c) is based upon a concentration of each of a plurality of nutrients and/or micronutrients present in the expressed breast milk and upon a plurality of infant factors, and wherein the desired level of concentration calculated in step (c) is defined as:
(i) a desired concentration of one or more of the plurality of nutrients and/or micronutrients; and/or
(ii) a desired ratio of two of the nutrients/micronutrients to one another.

### DETAILED DESCRIPTION

Before explaining at least one embodiment of the presently disclosed inventive concept(s) in detail, it is to be understood that the presently disclosed inventive concept(s) is not limited in its application to the details of construction and the arrangement of the components or steps or methodologies set forth in the following description or illustrated in the drawings.

Unless otherwise defined herein, technical terms used in connection with the presently disclosed inventive concept(s) shall have the meanings that are commonly understood by those of ordinary skill in the art. Further, unless otherwise required by context, singular terms shall include pluralities and plural terms shall include the singular.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. For example but not by way of limitation, when the term "about" is utilized, the designated value may vary by plus or minus twelve percent, or eleven percent, or ten percent, or nine percent, or eight percent, or seven percent, or six percent, or five percent, or four percent, or three percent, or two percent, or one percent. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z. The use of ordinal number terminology (i.e., "first," "second," "third," "fourth," etc.) is solely for the purpose of differentiating between two or more items and is not meant to imply any sequence or order or importance to one item over another or any order of addition, for example.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, and/or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, when associated with a particular event or circumstance, the term "substantially" means that the subsequently described event or circumstance occurs at least 80% of the time, or at least 85% of the time, or at least 90% of the time, or at least 95% of the time. The term "substantially adjacent" may mean that two items are 100% adjacent to one another, or that the two items are within close proximity to one another but not 100% adjacent to one another, or that a portion of one of the two items is not 100% adjacent to the other item but is within close proximity to the other item.

The terms "baby" and "infant" are used interchangeably herein and will be understood to include, but are not limited to, newborns, neonates, premature babies/infants (also referred to as preterm babies/infants or preemies), and low-birth-weight babies/infants.

The terms "human milk" and "breast milk" are used interchangeably herein and will be understood to include any form of milk, including, but not limited to, colostrum, foremilk, and hindmilk.

The terms "personalized human milk fortifier," "personalized, fortified concentrated human milk," and "personalized, concentrated human milk composition" are used interchangeably herein and will be understood to refer to a product formed of concentrated breast milk, wherein the breast milk is obtained from a mother of an infant to which the product is to be delivered.

The term "ultrafiltration" as used herein refers to a membrane-based filtration process that is use in industrial and biological settings to separate, purify, and concentrate macromolecular solutions. The term "selective ultrafiltration" refers to the selective concentration of one or more components of a fluid, and may further involve the separation and purification of said concentrated component(s) from one or more undesirable components. The term "selective ultrafiltration device" refers to a device capable of performing said selective separation, purification, and/or concentration of one or more components (i.e., nutrients and/or micronutrients) of a fluid, such as but not limited to, breast milk.

The term "associate" as used herein will be understood to refer to the direct or indirect connection of two or more items.

Turning now to the presently disclosed inventive concept(s), provided herein are devices, kits, and methods related to the need for premature and low-birth-weight babies to receive their own mother's milk. In certain embodiments, the presently disclosed inventive concept(s) is directed to devices, kits, and methods that allow each mother of a premature or low-birth-weight infant to concentrate her own milk and feed it to her infant, for example but not by way of limitation, during the first fragile days of life in the NICU. The benefits of these devices, kits, and methods include, but are not limited to: (1) emotional benefits -- parents will have less concerns, as the personalized human milk fortifier is delivering to the infant only his/her own mother's milk that has been directly expressed and concentrated; and (2) physical benefits -- the infants receive the goodness of their own mother's milk (which may even be provided at a specific concentration needed for their specific height, weight, and/or growth stage). Another advantage over the prior art is that there is no need for sterilization of the milk before feeding, and thus more nutrients/micronutrients/microflora are retained in the milk. In addition to providing a superior and personalized product over the prior art HMFs that are cow milk-based or human donor milk-based, the presently disclosed devices, kits, and methods provide a more cost effective product as well.

The presently disclosed inventive concept(s) possesses many benefits over the prior art. The goodness of a mother's own milk is provided to her premature or low-birth-weight infant by concentrating the milk to make personalized human milk fortifier that meets the personal nutritional needs of the premature or low-birth-weight infant while retaining the valuable microflora and nutrients/micronutrients present in the mother's own breast milk. There is no risk of donor milk transmitted diseases. In addition to providing additional probiotics and other beneficial microflora that are present in the expressed milk, the presently disclosed inventive concept(s) allows for selective concentration of specific nutrient(s) (such as, but not limited to, protein, fat, and carbohydrate); specific peptide(s); specific fatty acid(s); and/or specific micronutrient(s) (such as, but not limited to, calcium, phosphorous, potassium, sodium, and chloride) present in the expressed milk. Another advantageous feature of the presently disclosed inventive concept(s) is the selective removal of undesirable foreign substances from breast milk, such as but not limited to, nicotine, alcohol, medication(s), pharmaceutical by-product(s), pharmaceutical metabolite(s), chemical(s), certain bacteria, virus(es), and combinations thereof.

Certain embodiments of the presently disclosed inventive concept(s) are directed to devices that are used in the production of personalized human milk fortifiers. These devices include, but are not limited to, selective concentration devices that are capable of concentrating at least one nutrient and/or at least one micronutrient present in expressed breast milk from a mother of the infant. These devices will be described in further detail herein below. Certain embodiments of the presently disclosed inventive concept(s) are directed to kits useful for producing personalized human milk fortifiers. The kit may contain any combination of the below-described components/devices. In addition, the kit may further contain other component(s) and/or device(s) for performing any of the methods/processes described or otherwise contemplated herein. The nature of these additional component(s)/device(s) will depend upon the particular method/process format, and identification thereof is well within the skill of one of ordinary skill in the art.

In one embodiment of the presently disclosed inventive concept(s), a kit is provided for producing personalized human milk fortifier for an infant. The kit may include a selective concentration device that is capable of concentrating at least one nutrient and/or at least one micronutrient present in expressed breast milk from a mother of the infant. The selective concentration device may be capable of concentrating a plurality of nutrients and/or micronutrients. The kit may also include at least one graduated container (such as, but not limited to, a graduated cylinder) that is capable of receiving the expressed breast milk and that is capable of providing a measurement of a volume for the expressed breast milk. The graduated container(s) is also capable of association with the selective concentration device for concentration of the breast milk disposed therein to produce the personalized human milk fortifier.

The kit may further include an analyzer for determining a concentration of at least one nutrient and/or at least one micronutrient present in expressed breast milk. For example, but not by way of limitation, the analyzer may determine a concentration for each of a plurality of nutrients and/or micronutrients present in expressed breast milk. Calculation of specific concentrations present in each sample of breast milk is quite useful, since macronutrients in human milk can vary by day and even by the time of day.

Non-limiting examples of nutrients present in expressed breast milk for which a concentration may be determined (and for which concentration thereof may be desired) include protein, fat, and carbohydrate. Non-limiting examples of micronutrients present in expressed breast milk for which a concentration may be determined (and for which concentration thereof may be desired) include calcium, phosphorous, potassium, sodium, and chlorine.

In addition, the kit may further include a device that calculates a desired level of concentration of the breast milk, wherein the calculation is based upon at least two values: (1) a concentration of at least one nutrient and/or at least one micronutrient present in expressed breast milk, and (2) at least one infant factor. Any infant factors that may affect the amount of nutrients/micronutrients required for the infant and/or the volume in which the nutrients/micronutrients should be delivered may be utilized in accordance with the presently disclosed inventive concept(s). Examples of infant factors include, but are not limited to, an infant's height, an infant's weight, an infant's nutritional needs, an infant's fluid volume capacity, an infant's growth curve, and combinations thereof.

The device may calculate the desired level of concentration of the breast milk based upon a concentration of each of a plurality of nutrients and/or micronutrients present in expressed breast milk and a plurality of infant factors. When multiple nutrients/micronutrients/infant factors are considered, the desired level of concentration may be calculated as a desired concentration of one or more of the plurality of nutrients and/or micronutrients. Alternatively, the desired level of concentration may not be based on a specific value for a single nutrient/micronutrient, but may rather be calculated as a desired ratio of two of the nutrients/micronutrients to one another.

Any device capable of calculating one or more desired concentration values (or ratios) as described herein above -- and as based on the input of multiple variables -- may be utilized in accordance with the presently disclosed inventive concept(s). Non-limiting examples of devices that may be utilized include simple, manually manipulated devices (such as, but not limited to, slide rule-type devices, nomogram-type devices, sliding window(s)-type devices, as well as any other type of chart and/or diagram that requires manual manipulation to determine a calculated value) as well as logic devices (such as, but not limited to, software and/or hardware - type devices, such as a microprocessor, microcontroller, application specific integrated circuit, field programmable gate array, and the like).

For example, but not by way of limitation, the calculated desired concentration value(s) may be in a range of from about 1.1X to about 10X. In particular, non-limiting examples the calculated desired concentration value(s) may be in any range having (i) a low end value of any of about 1.1X, about 1.2X, about 1.3X, about 1.4X, about 1.5X, about 1.6X, about 1.7X, about 1.8X, about 1.9X, about 2.0X, about 2.1X, about 2.2X, about 2.3X, about 2.4X, about 2.5X, about 2.6X, about 2.7X, about 2.8X, about 2.9X, about 3.0X, about 3.1X, about 3.2X, about 3.3X, about 3.4X, about 3.5X, about 3.6X, about 3.7X, about 3.8X, about 3.9X, about 4.0X, about 4.1X, about 4.2X, about 4.3X, about 4.4X, about 4.5X, about 4.6X, about 4.7X, about 4.8X, about 4.9X, about 5.0X, about 5.1X, about 5.2X, about 5.3X, about 5.4X, about 5.5X, about 5.6X, about 5.7X, about 5.8X, about 5.9X, about 6.0X, about 6.1X, about 6.2X, about 6.3X, about 6.4X, about 6.5X, about 6.6X, about 6.7X, about 6.8X, about 6.9X, about 7.0X, about 7.1X, about 7.2X, about 7.3X, about 7.4X, about 7.5X, about 7.6X, about 7.7X, about 7.8X, about 7.9X, about 8.0X, about 8.1X, about 8.2X, about 8.3X, about 8.4X, about 8.5X, about 8.6X, about 8.7X, about 8.8X, about 8.9X, about 9.0X, and the like; and (ii) a high end value of any of about 2.0X, about 2.1X, about 2.2X, about 2.3X, about 2.4X, about 2.5X, about 2.6X, about 2.7X, about 2.8X, about 2.9X, about 3.0X, about 3.1X, about 3.2X, about 3.3X, about 3.4X, about 3.5X, about 3.6X, about 3.7X, about 3.8X, about 3.9X, about 4.0X, about 4.1X, about 4.2X, about 4.3X, about 4.4X, about 4.5X, about 4.6X, about 4.7X, about 4.8X, about 4.9X, about 5.0X, about 5.1X, about 5.2X, about 5.3X, about 5.4X, about 5.5X, about 5.6X, about 5.7X, about 5.8X, about 5.9X, about 6.0X, about 6.1X, about 6.2X, about 6.3X, about 6.4X, about 6.5X, about 6.6X, about 6.7X, about 6.8X, about 6.9X, about 7.0X, about 7.1X, about 7.2X, about 7.3X, about 7.4X, about 7.5X, about 7.6X, about 7.7X, about 7.8X, about 7.9X, about 8.0X, about 8.1X, about 8.2X, about 8.3X, about 8.4X, about 8.5X, about 8.6X, about 8.7X, about 8.8X, about 8.9X, about 9.0X, about 9.1X, about 9.2X, about 9.3X, about 9.4X, about 9.5X, about 9.6X, about 9.7X, about 9.8X, about 9.9X, about 10X, and the like.

In certain embodiments, the kit may further include at least one feeding apparatus in which the personalized human milk fortifier may be disposed and that may be used for delivery of the personalized human milk fortifier to the infant. In one particular, non-limiting embodiment, the feeding apparatus may be a feeding syringe, so as to avoid nipple confusion (especially if the mother is breastfeeding or plans to breastfeed upon discharge).

In certain embodiments, the selective concentration device may be a selective ultrafiltration device and thus may be capable of removing at least one undesirable substance from the expressed breast milk. In other embodiments, the kit may include a separate filtration device for removing at least one undesirable substance from the expressed breast milk. Examples of undesirable substances that may potentially be present in breast milk include, but are not limited to, nicotine, alcohol, medications, pharmaceutical by-products, pharmaceutical metabolites, chemicals, certain bacteria, viruses, and combinations thereof.

The kits of the presently disclosed inventive concept(s) may further include one or more sets of instructions. The instructions may explain how to use the kit, how to deliver the personalized human milk fortifier to the infant, and/or how to store the personalized human milk fortifier. Non-limiting, exemplary storage periods that may be provided in the instructions include storage under refrigerated conditions for a period of up to five days and storage under frozen conditions for a period of up to three months.

Any type of format capable of conveying the desired information (and/or directing a user's attention to a location where said information can be found) may be utilized as the instructions described or otherwise contemplated herein. Non-limiting examples of formats in which the instructions may be provided include written wording and/or pictorial drawings, a website address, a bar code (such as but not limited to, a QR code) that is readable by an imaging device/code reader, combinations thereof, and the like.

Any of the kits described herein above or otherwise contemplated herein may be used with any of the methods described or otherwise contemplated herein below.

Certain other embodiments of the presently disclosed inventive concept(s) are directed to a method of producing a personalized human milk fortifier for an infant. In the method, expressed breast milk is obtained from a mother of the infant, and a portion thereof is analyzed to determine a concentration of at least one nutrient and/or at least one micronutrient present therein. A desired level of concentration of the expressed breast milk is then calculated based upon: (1) the concentration of the at least one nutrient and/or at least one micronutrient present in the expressed breast milk, and (2) at least one infant factor (as described in detail herein above). The expressed breast milk is then exposed to a selective concentration device that concentrates the at least one nutrient and/or the at least one micronutrient (that was analyzed as described herein above) to the desired level of concentration as calculated above, thereby producing the personalized human milk fortifier that is specific for the infant and obtained directly from the milk of the infant's mother.

The method may further include the step of administering at least a portion of the personalized human milk fortifier to the infant. Alternatively and/or in addition thereto, the method may further include the step of storing the personalized human milk fortifier under refrigerated or frozen conditions for a period of time.

In particular, non-limiting embodiments of the presently disclosed inventive concept(s), a concentration of a plurality of nutrients and/or micronutrients present is the expressed breast milk is determined; in this embodiment, the selective concentration device concentrates the plurality of nutrients and/or micronutrients as described above. When the calculated level of concentration for the breast milk is based upon a concentration of each of a plurality of nutrients and/or micronutrients present in the expressed breast milk as well as a plurality of infant factors, the calculated level of concentration may include: (i) a desired specific concentration of one or more of the plurality of nutrients and/or micronutrients, and/or (ii) a desired ratio of two of the nutrients/micronutrients to one another.

In certain embodiments, the method may further include the step of filtering the expressed breast milk to remove at least one undesirable substance (such as, but not limited to, nicotine, alcohol, medications, pharmaceutical by-products, pharmaceutical metabolites, chemicals, certain bacteria, viruses, and combinations thereof) from the expressed breast milk. This filtration step may be performed by the selective concentration device if the selective concentration device is a selective ultrafiltration device. Alternatively, a separate filtration device may be utilized to perform this step.

The methods of the presently disclosed inventive concept(s) may be performed in the absence of any sterilization steps, thus retaining substantially all desired components of the expressed breast milk.

The combinations of method steps described herein above may be performed simultaneously or wholly or partialy sequentially. In addition, the exemplary sequences of method steps provided herein above are for the purposes of illustration only; it will be understood that the individual steps, as well as the particular order of steps, may vary, and the sequence of steps may be performed in any order, so long as the materials and packages described herein are capable of functioning in accordance with the presently disclosed inventive concept(s).

### EXAMPLES

Examples are provided hereinbelow. However, the presently disclosed inventive concept(s) is to be understood to not be limited in its application to the specific experimentation, results, and procedures described herein after. Rather, the Examples are simply provided as one of various embodiments and are meant to be exemplary, not exhaustive.

### Example 1

Figure 1 is a schematic flow chart illustrating one embodiment of a method constructed in accordance with the presently disclosed inventive concept(s).

### Example 2

In one example of a method constructed in accordance with the presently disclosed inventive concept(s), a small scale, selective ultrafiltration procedure is used so that a mother's own expressed milk is concentrated to a level needed based on the infant's nutritional needs and daily liquid volume capacity. A human milk analyzer can be used just prior to concentrating the milk, since macronutrients in human milk vary by day and daypart, to determine the exact level of concentration. A software program can be provided, and a healthcare professional (HCP) can enter in the results of the human milk analysis and the desired nutrition and/or liquid volume (i.e., in milliliters). The software program may calculate the level of concentration needed.

If the concentrated milk is not needed immediately, it can be refrigerated or frozen for a period of time. For example but not by way of limitation, the concentrated milk can be refrigerated for up to 5 days or frozen for up to 3 months. Defrosting should be done overnight in the fridge or in a warm water bath for no longer than 15 minutes. Any unused milk should be discarded after warming in water bath.

**Table I. Selective Concentration Example**

| | **Pumped Breast Milk** | **Pumped Breast Milk + Liquid HMF** | **Selective Concentration needed for Concentrated Breast Milk** |
|---|---|---|---|
| Energy kcal | | | |
| Protein (g) | 1.6 | 3.2 | 2X |
| Fat (g) | 4.2 | 4.8 | 1.1X |
| Carbohydrate (g) | 7 | 6.6 | 1X |
| Calcium (mg) | 28 | 118 | 4.2X |
| Phosphorous (mg) | 14 | 65 | 4.6X |

### Example 3

A non-limiting example of a kit constructed in accordance with the presently disclosed inventive concept(s) includes the following components: (a) a small scale ultrafiltration machine for liquid volumes between 2-16 fluid ounces; (b) graduated cylinders - small (2-8 oz.) cylinder(s) and/or large (8-16 oz.) cylinder(s); (c) graduated cylinder caps for refrigeration or frozen storage of the personalized human milk fortifier in the graduated cylinder(s); and (d) a software program (or other device) to calculate the level of human milk concentration needed by ultrafiltration.

Other components that may be present in the kit include the following: (a) a breast milk analyzer for determining content of various nutrients (i.e., protein, fat, carbohydrate, etc.) and/or micronutrients (i.e., calcium, phosphorous, etc.) present in the breast milk; and (b) feeding syringe(s).

For example, approximately 8 oz. of fluid milk is poured into a graduated cylinder and placed into an ultrafiltration unit. The level of concentration is entered, and the start button on the ultrafiltration unit is pushed. Within seconds, the mother's milk is concentrated and is ready to be fed to the infant by any known method. One specific, non-limiting feeding method uses a syringe, so as to avoid nipple confusion (especially if the mother is breastfeeding or plans to breastfeed upon discharge).

Thus, in accordance with the presently disclosed inventive concept(s), there have been provided methods of producing a personalized human milk fortifier, as well as devices and kits related to same, that fully satisfy the objectives and advantages set forth hereinabove. Although the presently disclosed inventive concept(s) has been described in conjunction with the specific drawings, experimentation, results, and language set forth hereinabove, it is evident that many alternatives, modifications, and variations will be apparent to those skilled in the art. Accordingly, it is intended to embrace all such alternatives, modifications, and variations that fall within the spirit and broad scope of the presently disclosed inventive concept(s).

## Claims

1. A kit for producing a personalized, concentrated human milk composition for an infant, the kit comprising:
at least one analyzer for determining a concentration of at least one nutrient and/or at least one micronutrient present in the expressed breast milk from the mother;
at least one selective concentration device capable of concentrating at least one nutrient and/or at least one micronutrient present in expressed breast milk from a mother of the infant and wherein the selective concentration device is a selective ultrafiltration device;
at least one graduated container capable of receiving the expressed breast milk and capable of measuring a volume of the expressed breast milk, the at least one graduated container being capable of association with the selective concentration device for concentration of the breast milk disposed therein to produce the personalized, concentrated human milk composition;
and at least one device that calculates a desired level of concentration of the breast milk based upon a concentration of at least one nutrient and/or at least one micronutrient present in the expressed breast milk and at least one infant factor selected from the group consisting of the infant's height, the infant's weight, the infant's nutritional needs, the infant's fluid volume capacity, the infant's growth curve, and combinations thereof and wherein the device calculates a desired level of concentration of the breast milk based upon a concentration of each of a plurality of nutrients and/or micronutrients present in the expressed breast milk and a plurality of infant factors, and wherein the calculated level of concentration is defined as:
(i) a desired concentration of one or more of the plurality of nutrients and/or micronutrients; and/or
(ii) a desired ratio of two of the nutrients/micronutrients to one another.

2. The kit of claim 1, wherein the selective concentration device is capable of concentrating a plurality of nutrients and/or micronutrients.

3. The kit of any of claims 1-2, wherein the analyzer determines a concentration for each of a plurality of nutrients and/or micronutrients present in the expressed breast milk.

4. The kit of any of claims 1-3, wherein each nutrient is selected from the group consisting of protein, fat, and carbohydrate, and wherein each micronutrient is selected from the group consisting of calcium, phosphorous, potassium, sodium, and chloride.

5. The kit of any of claims 1-4, further comprising at least one feeding apparatus in which the personalized, concentrated human milk composition may be disposed for delivery to the infant.

6. The kit of any of claims 1-5, wherein the selective ultrafiltration device is capable of removing at least one undesirable substance from the expressed breast milk.

7. The kit of any of claims 1-6, further comprising at least one filtration device for removing at least one undesirable substance from the expressed breast milk.

8. The kit of claim 6 or 7, wherein the at least one undesirable substance is selected from the group consisting of nicotine, alcohol, medications, pharmaceutical by-products, pharmaceutical metabolites, chemicals, certain bacteria, viruses, and combinations thereof.

9. The kit of any of claims 1-8, further comprising instructions, wherein the instructions are selected from at least one of:
(a) instructions for use of the kit;
(b) instructions for delivering at least a portion of the personalized, concentrated human milk composition to the infant; and
(c) instructions for storage of at least a portion of the personalized, concentrated human milk composition for a period of up to five days under refrigerated conditions or for a period of up to six months under frozen conditions.

10. A method of producing a personalized, concentrated human milk composition for an infant, the method comprising the steps of:
(a) obtaining expressed breast milk from a mother of an infant;
(b) analyzing a portion of the expressed breast milk to determine a concentration of at least one nutrient and/or at least one micronutrient present therein;
(c) calculating a desired level of concentration of the expressed breast milk based upon the concentration of the at least one nutrient or micronutrient present in the expressed breast milk and at least one infant factor selected from the group consisting of the infant's height, the infant's weight, the infant's nutritional needs, the infant's fluid volume capacity, the infant's growth curve, and combinations thereof; and
(d) exposing the expressed breast milk to a selective concentration device which is a selective ultrafiltration device; that concentrates the at least one nutrient and/or the at least one micronutrient analyzed in step (b) to the desired level of concentration calculated in step (c), thereby producing the personalized, concentrated human milk composition;
And wherein, in step (b), a concentration of a plurality of nutrients and/or micronutrients present is the expressed breast milk is determined, and wherein, in step (d), the selective concentration device concentrates the plurality of nutrients and/or micronutrients
wherein preferably the desired level of concentration calculated in step (c) is based upon a concentration of each of a plurality of nutrients and/or micronutrients present in the expressed breast milk and upon a plurality of infant factors, and wherein the desired level of concentration calculated in step (c) is defined as:
(i) a desired concentration of one or more of the plurality of nutrients and/or micronutrients; and/or
(ii) a desired ratio of two of the nutrients/micronutrients to one another.

11. The method of claim 10, further comprising the step of storing at least a portion of the personalized, concentrated human milk composition under refrigerated or frozen conditions for a period of time.

12. The method of claim 10 or 11, wherein at least a portion of the personalized, concentrated human milk composition is for administration to the infant.

13. The method of claim any of claims 10-12, wherein each nutrient is selected from the group consisting of protein, fat, and carbohydrate, and wherein each micronutrient is selected from the group consisting of calcium, phosphorous, potassium, sodium, and chloride.

14. The method of any of claims 10-13, further comprising the step of filtering the expressed breast milk to remove at least one undesirable substance from the expressed breast milk.
wherein preferably the at least one undesirable substance is selected from the group consisting of nicotine, alcohol, medications, pharmaceutical by-products, pharmaceutical metabolites, chemicals, certain bacteria, viruses, and combinations thereof.

## Patentansprüche

1. Kit zum Herstellen einer personalisierten, konzentrierten menschlichen Milchzusammensetzung für einen Säugling, wobei das Kit Folgendes umfasst:
mindestens ein Analysegerät zum Bestimmen einer Konzentration von mindestens einem Nährstoff und/oder mindestens einem Mikronährstoff, die in der abgegebenen Muttermilch von der Mutter vorhanden sind;
mindestens eine selektive Konzentrationsvorrichtung, die zum Konzentrieren von mindestens einem Nährstoff und/oder mindestens einem Mikronährstoff, die in der abgegebenen Muttermilch von einer Mutter des Säuglings vorhanden sind, in der Lage ist und wobei die selektive Konzentrationsvorrichtung eine selektive Ultrafiltrationsvorrichtung ist;
mindestens einen abgestuften Behälter, der zum Aufnehmen der abgegebenen Muttermilch in der Lage ist und zum Messen eines Volumens der abgegebenen Muttermilch in der Lage ist, wobei der mindestens eine abgestufte Behälter zur Verbindung mit der selektiven Konzentrationsvorrichtung zur Konzentration der darin angeordneten Muttermilch in der Lage ist, um die personalisierte, konzentrierte menschliche Milchzusammensetzung herzustellen;
und mindestens eine Vorrichtung, die einen gewünschten Konzentrationsspiegel der Muttermilch basierend auf einer Konzentration von mindestens einem Nährstoff und/oder mindestens einem Mikronährstoff, die in der abgegebenen Muttermilch vorhanden sind, und mindestens einem Säuglingsfaktor berechnet, der aus der Gruppe ausgewählt ist, bestehend aus der Größe des Säuglings, dem Gewicht des Säuglings, dem Nährstoffbedarf des Säuglings, der Fluidvolumenkapazität des Säuglings, der Wachstumskurve des Säuglings und Kombinationen davon, und wobei die Vorrichtung einen gewünschten Konzentrationsspiegel der Muttermilch basierend auf einer Konzentration von jedem einer Vielzahl von Nährstoffen und/oder Mikronährstoffen, die in der abgegebenen Muttermilch vorhanden ist, und einer Vielzahl von Säuglingsfaktoren berechnet, und wobei der berechnete Konzentrationsspiegel wie folgt definiert ist:
(i) eine gewünschte Konzentration von einem oder mehreren der Vielzahl von Nährstoffen und/oder Mikronährstoffen; und/oder
(ii) ein gewünschtes Verhältnis von zwei der Nährstoffe/Mikronährstoffe zueinander.

2. Kit nach Anspruch 1, wobei die selektive Konzentrationsvorrichtung zum Konzentrieren einer Vielzahl von Nährstoffen und/oder Mikronährstoffen in der Lage ist.

3. Kit nach einem der Ansprüche 1 bis 2, wobei das Analysegerät eine Konzentration für jeden von einer Vielzahl von Nährstoffen und/oder Mikronährstoffen bestimmt, die in der abgegebenen Muttermilch vorhanden sind.

4. Kit nach einem der Ansprüche 1 bis 3, wobei jeder Nährstoff aus der Gruppe ausgewählt ist, bestehend aus Protein, Fett und Kohlenhydrat, und wobei jeder Mikronährstoff aus der Gruppe ausgewählt ist, bestehend aus Kalzium, Phosphor, Kalium, Natrium und Chlorid.

5. Kit nach einem der Ansprüche 1 bis 4, ferner umfassend mindestens eine Fütterungseinrichtung, in der die personalisierte, konzentrierte menschliche Milchzusammensetzung zur Abgabe an den Säugling angeordnet werden kann.

6. Kit nach einem der Ansprüche 1 bis 5, wobei die selektive Ultrafiltrationsvorrichtung zum Entfernen von mindestens einer unerwünschten Substanz aus der abgegebenen Muttermilch in der Lage ist.

7. Kit nach einem der Ansprüche 1 bis 6, ferner umfassend mindestens eine Filtrationsvorrichtung zum Entfernen von mindestens einer unerwünschten Substanz aus der abgegebenen Muttermilch.

8. Kit nach Anspruch 6 oder 7, wobei die mindestens eine unerwünschte Substanz aus der Gruppe ausgewählt ist, bestehend aus Nikotin, Alkohol, Medikamenten, pharmazeutischen Nebenprodukten, pharmazeutischen Stoffwechselprodukten, Chemikalien, bestimmten Bakterien, Viren und Kombinationen davon.

9. Kit nach einem der Ansprüche 1 bis 8, ferner umfassend Anweisungen, wobei die Anweisungen aus mindestens einem von Folgenden ausgewählt sind:
(a) Anweisungen zur Verwendung des Kits;
(b) Anweisungen zum Abgeben von mindestens einem Teil der personalisierten, konzentrierten menschlichen Milchzusammensetzung an den Säugling; und
(c) Anweisungen zur Lagerung von mindestens einem Teil der personalisierten, konzentrierten Humanmilchzusammensetzung über einen Zeitraum von bis zu fünf Tagen unter gekühlten Bedingungen oder über einen Zeitraum von bis zu sechs Monaten unter gefrorenen Bedingungen.

10. Verfahren zum Herstellen einer personalisierten, konzentrierten menschlichen Milchzusammensetzung für einen Säugling, wobei das Verfahren die folgenden Schritte umfasst:
(a) Erhalten von abgegebener Muttermilch von einer Mutter eines Säuglings;
(b) Analysieren eines Teils der abgegebenen Muttermilch, um eine Konzentration von mindestens einem Nährstoff und/oder mindestens einem Mikronährstoff, die darin vorhanden sind, zu bestimmen;
(c) Berechnen eines gewünschten Konzentrationsspiegels der abgegebenen Muttermilch basierend auf der Konzentration des mindestens einen Nährstoffs und/oder Mikronährstoffs, die in der abgegebenen Muttermilch vorhanden sind, und mindestens einem Säuglingsfaktor, der aus der Gruppe ausgewählt ist, bestehend aus der Größe des Säuglings, dem Gewicht des Säuglings, dem Nährstoffbedarf des Säuglings, der Fluidvolumenkapazität des Säuglings, der Wachstumskurve des Säuglings und Kombinationen davon; und
(d) Aussetzen der abgegebenen Muttermilch einer selektiven Konzentrationsvorrichtung, die eine selektive Ultrafiltrationsvorrichtung ist; die den mindestens einen Nährstoff und/oder den mindestens einen Mikronährstoff, die in Schritt (b) analysiert wurden, auf den gewünschten Konzentrationsspiegel konzentriert, der in Schritt (c) berechnet wurde, wodurch die personalisierte, konzentrierte menschliche Milchzusammensetzung hergestellt wird;
und wobei in Schritt (b) eine Konzentration von einer Vielzahl von Nährstoffen und/oder Mikronährstoffen, die in der abgegebenen Muttermilch vorhanden sind, bestimmt wird, und wobei die selektive Konzentrationsvorrichtung in Schritt (d) die Vielzahl von Nährstoffen und/oder Mikronährstoffen konzentriert, wobei der gewünschte Konzentrationsspiegel, der in Schritt (c) berechnet wurde, auf einer Konzentration von jedem von einer Vielzahl von Nährstoffen und/oder Mikronährstoffen, die in der abgegebenen Muttermilch vorliegen, und auf einer Vielzahl von Säuglingsfaktoren basiert, und wobei der Konzentrationsspiegel, der in Schritt (c) berechnet wurde, wie folgt definiert ist:
(i) eine gewünschte Konzentration von einem oder mehreren der Vielzahl von Nährstoffen und/oder Mikronährstoffen; und/oder
(ii) ein gewünschtes Verhältnis von zwei der Nährstoffe/Mikronährstoffe zueinander.

11. Verfahren nach Anspruch 10, ferner umfassend den Schritt zum Lagern von mindestens einem Teil der personalisierten, konzentrierten menschlichen Milchzusammensetzung unter gekühlten oder gefrorenen Bedingungen über einen Zeitraum.

12. Verfahren nach Anspruch 10 oder 11, wobei mindestens ein Teil der personalisierten, konzentrierten menschlichen Milchzusammensetzung zur Verabreichung an den Säugling gedacht ist.

13. Verfahren nach einem der Ansprüche 10 bis 12, wobei jeder Nährstoff aus der Gruppe ausgewählt ist, bestehend aus Protein, Fett und Kohlenhydrat, und wobei jeder Mikronährstoff aus der Gruppe ausgewählt ist, bestehend aus Kalzium, Phosphor, Kalium, Natrium und Chlorid.

14. Verfahren nach einem der Ansprüche 10 bis 13, ferner umfassend den Schritt zum Filtern der Muttermilch, um mindestens eine unerwünschte Substanz aus der abgegebenen Muttermilch zu entfernen,
wobei die mindestens eine unerwünschte Substanz vorzugsweise aus der Gruppe ausgewählt ist, bestehend aus Nikotin, Alkohol, Medikamenten, pharmazeutischen Nebenprodukten, pharmazeutischen Stoffwechselprodukten, Chemikalien, bestimmten Bakterien, Viren und Kombinationen davon.

## Revendications

1. Kit pour produire une composition de lait humain concentré personnalisé pour un nourrisson, le kit comprenant :
au moins un analyseur pour déterminer une concentration d'au moins un nutriment et/ou d'au moins un micronutriment présent dans le lait maternel exprimé provenant de la mère ;
au moins un dispositif de concentration sélective capable de concentrer au moins un nutriment et/ou au moins un micronutriment présent dans le lait maternel exprimé provenant de la mère du nourrisson et dans lequel le dispositif de concentration sélective est un dispositif d'ultrafiltration sélective ;
au moins un récipient gradué pouvant recevoir le lait maternel exprimé et pouvant mesurer un volume du lait maternel exprimé, l'au moins un récipient gradué pouvant être associé au dispositif de concentration sélective pour la concentration du lait maternel disposé à l'intérieur pour produire la composition de lait humain concentré personnalisée ;
et au moins un dispositif qui calcule un niveau souhaité de concentration du lait maternel sur la base d'une concentration d'au moins un nutriment et/ou d'au moins un micronutriment présent dans le lait maternel exprimé et d'au moins un facteur du nourrisson choisi dans le groupe constitué par la taille du nourrisson, le poids du nourrisson, les besoins nutritionnels du nourrisson, la capacité volumique des fluides du nourrisson, la courbe de croissance du nourrisson, et leurs combinaisons, et dans lequel le dispositif calcule un niveau souhaité de concentration du lait maternel sur la base d'une concentration de chacun d'une pluralité de nutriments et/ou de micronutriments présents dans le lait maternel exprimé et d'une pluralité de facteurs du nourrisson, et dans lequel le niveau calculé de concentration est défini comme suit :
(i) une concentration souhaitée d'un ou plusieurs de la pluralité de nutriments et/ou de micronutriments ; et/ou
(ii) un rapport souhaité entre les deux nutriments/micronutriments.

2. Kit selon la revendication 1, dans lequel le dispositif de concentration sélective est capable de concentrer une pluralité de nutriments et/ou de micronutriments.

3. Kit selon l'une quelconque des revendications 1 à 2, dans lequel l'analyseur détermine une concentration pour chacun d'une pluralité de nutriments et/ou de micronutriments présents dans le lait maternel exprimé.

4. Kit selon l'une quelconque des revendications 1 à 3, dans lequel chaque nutriment est choisi dans le groupe constitué par des protéines, des matières grasses et des glucides, et dans lequel chaque micronutriment est choisi dans le groupe constitué par du calcium, du phosphore, du potassium, du sodium et du chlorure.

5. Kit selon l'une quelconque des revendications 1 à 4, comprenant en outre au moins un appareil d'alimentation dans lequel la composition de lait humain concentré personnalisée peut être disposée pour être délivrée au nourrisson.

6. Kit selon l'une quelconque des revendications 1 à 5, dans lequel le dispositif d'ultrafiltration sélective est capable d'éliminer au moins une substance indésirable du lait maternel exprimé.

7. Kit selon l'une quelconque des revendications 1 à 6, comprenant en outre au moins un dispositif de filtration pour éliminer au moins une substance indésirable du lait maternel exprimé.

8. Kit selon la revendication 6 ou 7, dans lequel l'au moins une substance indésirable est choisie dans le groupe constitué par de la nicotine, de l'alcool, des médicaments, des sous-produits pharmaceutiques, des métabolites pharmaceutiques, des produits chimiques, certaines bactéries, des virus et leurs combinaisons.

9. Kit selon l'une quelconque des revendications 1 à 8, comprenant en outre des instructions, dans lequel les instructions sont sélectionnées parmi au moins l'une des suivantes :
(a) instructions d'utilisation du kit ;
(b) instructions pour fournir au moins une partie de la composition du lait humain concentré personnalisée au nourrisson ; et
(c) instructions pour le stockage d'au moins une partie de la composition de lait humain concentré personnalisée pendant une période allant jusqu'à cinq jours dans des conditions réfrigérées ou pendant une période allant jusqu'à six mois dans des conditions congelées.

10. Procédé de production d'une composition de lait humain concentré personnalisé pour un nourrisson, le procédé comprenant les étapes suivantes :
(a) obtention d'un lait maternel exprimé provenant de la mère d'un nourrisson ;
(b) analyse d'une partie du lait maternel exprimé pour déterminer une concentration d'au moins un nutriment et/ou d'au moins un micronutriment présent dans celui-ci ;
(c) calcul d'un niveau souhaité de concentration du lait maternel exprimé sur la base de la concentration de l'au moins un nutriment ou micronutriment présent dans le lait maternel exprimé et d'au moins un facteur du nourrisson choisi dans le groupe constitué par la taille du nourrisson, le poids du nourrisson, les besoins nutritionnels du nourrisson, la capacité volumique des fluides du nourrisson, la courbe de croissance du nourrisson, et leurs combinaisons ; et
(d) exposition du lait maternel exprimé à un dispositif de concentration sélective qui est un dispositif d'ultrafiltration sélective ; qui concentre l'au moins un nutriment et/ou l'au moins un micronutriment analysé à l'étape (b) au niveau de concentration souhaité calculé à l'étape (c), produisant ainsi la composition de lait humain concentré personnalisée ;
Et dans lequel, à l'étape (b), une concentration d'une pluralité de nutriments et/ou de micronutriments présents dans le lait maternel exprimé est déterminée, et dans lequel, à l'étape (d), le dispositif de concentration sélective concentre la pluralité de nutriments et/ou de micronutriments,
dans lequel, de préférence, le niveau de concentration souhaité calculé à l'étape (c) est basé sur une concentration de chacun d'une pluralité de nutriments et/ou de micronutriments présents dans le lait maternel exprimé et sur une pluralité de facteurs du nourrisson, et dans lequel le niveau de concentration souhaité calculé à l'étape (c) est défini comme suit :
(i) une concentration souhaitée d'un ou plusieurs de la pluralité de nutriments et/ou de micronutriments ; et/ou
(ii) un rapport souhaité entre les deux nutriments/micronutriments.

11. Procédé selon la revendication 10, comprenant en outre l'étape de stockage d'au moins une partie de la composition de lait humain concentré personnalisée, dans des conditions réfrigérées ou congelées pendant une certaine période.

12. Procédé selon la revendication 10 ou 11, dans lequel au moins une partie de la composition de lait humain concentré personnalisée est destinée à être administrée au nourrisson.

13. Procédé selon l'une quelconque des revendications 10 à 12, dans lequel chaque nutriment est choisi dans le groupe constitué par des protéines, des matières grasses et des glucides, et dans lequel chaque micronutriment est choisi dans le groupe constitué par du calcium, du phosphore, du potassium, du sodium et du chlorure.

14. Procédé selon l'une quelconque des revendications 10 à 13, comprenant en outre l'étape consistant à filtrer le lait maternel exprimé pour éliminer au moins une substance indésirable du lait maternel exprimé.
dans lequel, de préférence, l'au moins une substance indésirable est choisie dans le groupe constitué par de la nicotine, de l'alcool, des médicaments, des sous-produits pharmaceutiques, des métabolites pharmaceutiques, des produits chimiques, certaines bactéries, des virus et leurs combinaisons.
